# EUROPÄISCHE PATENTSCHRIFT

(11) **EP 1 428 484 B1**
(45) Veröffentlichungstag und Bekanntmachung des Hinweises auf die Patenterteilung: **30.04.2008**
(21) Anmeldenummer: 02406079.0
(22) Anmeldetag: 09.12.2002
(51) Int. Cl.: A61F 2/16

(54) **Kolbennadel für einen intraokularen Linseninjektor**
Plunger nedle for iol injector
Brin de piston pour un injecteur de lentilles intraoculaires

(43) Veröffentlichungstag der Anmeldung: 16.06.2004
(73) Patentinhaber: Anton Meyer & Co. AG, 2562 Port (CH); Asico LLC, Westmont, IL 60559 (US)
(72) Erfinder: Meyer, Rolf, CH - 2562 Port (CH)
(74) Vertreter: Clerc, Natalia

(56) Entgegenhaltungen:
- WO-A-02/074208
- US-A- 6 010 510
- US-A- 6 022 358

## Beschreibung

### Technisches Gebiet

Die Erfindung betrifft eine Kolbennadel für einen intraokularen Linsen-Injektor gemäss Oberbegriff des Patentanspruchs 1.

### Stand der Technik

Trübe natürliche Augenlinsen lassen sich durch künstliche Linsen, sogenannte IOL's, ersetzen. Bei diesem Eingriff wird zuerst die trübe Augenlinse des Patienten entfernt. Die künstliche Linse wird anschliessend entweder von Hand mit Hilfe von Pinzetten oder mit Hilfe eines Injektors eingesetzt. Dieser Injektor besteht im wesentlichen aus einem hülsenförmigen Griffkörper mit einer Linsenaufnahme und einem im Griffkörper geführt verschiebbaren Kolben. Die Linse wird im gefalteten Zustand in einer Kartusche verpackt in die Linsenaufnahme eingesetzt. Ähnlich wie bei einer Spritze wird die Linse durch eine Durchlassöffnung in das Auge injiziert. Dabei wird der Kolben entweder geradlinig nach vorne gestossen oder in einem Gewinde geführt nach vorne gedreht. Im Auge kann sich die Linse entfalten, wobei die Entfaltung oft durch Drehung des Kolbens unterstützt wird.

Im Stand der Technik sind verschiedene Injektoren bekannt. Eine entsprechende Auflistung der bekanntesten Arten ist in der Beschreibungseinleitung der US 6'010'510 aufgeführt. Ferner ist EP-A-1 287 791 der Anmelderin ein weiterer Injektor beschrieben. Diese Patentschrift ist Teil des Standes der Technik unter Artikel 54(3) EPÜ.

Werden derartige Injektoren verwendet, so muss darauf geachtet werden, dass der Kolben die künstliche Linse ohne Schaden durch die Durchlassöffnung stossen kann. Der Kolben weist deshalb eine Kolbennadel mit einer Kolbenspitze auf, welche so zu formen ist, dass ein schadloses Injizieren der Linse möglich ist. Zudem soll verhindert werden, dass die Linse zu irgendeinem Zeitpunkt auf unerwünschte Art und Weise um die Kolbenspitze herumfliesst und im Injektor verklemmt.

Erschwert wird das schadlose Einführen der Linse durch allfällige an der Linse angebrachte Verankerungsmittel, sogenannte Haptics. Da diese von der gefalteten Linse abstehen, können sie leicht zwischen dem Kolben und der inneren Injektorwand eingeklemmt und abgerissen werden.

Die bisher bekannten Kolbennadeln erfüllen diese Bedingungen nur teilweise oder lassen sich nur in speziell angepassten Injektoren einsetzen.

So beschreiben US 5'643'276 und US 6'447'520, deren Familienmitglied WO 02/074208 die Präambel des Anspruchs 1 definiert, Kolbennadeln mit einer gabelförmigen Kolbenspitze, in welcher die Linse eingeklemmt werden kann.

US 6'010'510 offenbart eine Kolbennadel mit einer Kolbenspitze, welche zungenförmig ausgestaltet ist. Die Kolbenspitze verläuft dabei versetzt zur Längsachse des Kolbens. Dadurch soll gewährleistet werden, dass die Kolbenspitze beim Vorschieben gegen den Boden der Kartusche gedrückt wird. Die Kolbenspitze soll dadurch nicht über die Linse geschoben werden können, so dass sich die Linse nicht auf unerwünschte Weise um die Kolbennadel legen kann. Damit dies jedoch erreicht werden kann, muss die Kartusche innenseitig eine Stufe aufweisen, damit die Kolbenspitze unter die Linse gleiten kann.

US 6'241'737 zeigt eine facettenartige Kolbenspitze mit einer konkaven Stirnfläche zur Kontaktierung der Linse. Diese Kolbenspitze ist relativ komplex und entsprechend schwierig zu fertigen. Zudem weist sie eine Vielzahl an Kanten auf, was die Gefahr einer Beschädigung der Linse erhöht.

### Darstellung der Erfindung

Es ist deshalb Aufgabe der Erfindung, eine Kolbennadel für einen intraokularen Linsen-Injektor zu schaffen, welcher ein schadloses Ausstossen einer gefalteten Linse ermöglicht und eine Verklemmung innerhalb des Injektors vermeidet.

Diese Aufgabe löst eine Kolbennadel mit den Merkmalen des Patentanspruchs 1.

Die erfindungsgemässe Kolbennadel weist eine Kolbenspitze auf, deren vordere Stirnfläche, welche den Kontaktierungsbereich bildet, zylinderförmig gekrümmt ist. Mit anderen Worten, die Stirnfläche ist wie ein negatives Abbild eines Teils einer Mantelfläche eines Zylinders, insbesondere eines Kreiszylinders, gekrümmt. In einer Seitenansicht ist die Stirnfläche nach innen gekrümmt, wobei sich diese Krümmung entlang der zu dieser Ansicht senkrechten Achse wie bei einem Zylinder gleichbleibend wiederholt.

Diese Kolbennadel lässt sich relativ einfach herstellen. sie weist im vorderen Bereich ein minimales Mass an Kanten auf, so dass die Linse nicht verletzt wird. Zudem lässt sie sich in unterschiedlichen Injektortypen und für unterschiedliche Linsentypen verwenden. Vorzugsweise ist jedoch der Krümmungsradius der Stirnfläche der Kolbenspitze dem jeweiligen Radius der Linse angepasst, so dass die Stirnfläche optimal an der gefalteten Linse anliegt und diese gleichmässig geführt aus dem Injektor ausstossen kann. Die Länge der Krümmung, hier mit dem Öffnungswinkel des Zylindersegmentes bezeichnet, ist vorzugsweise dem Faltungsgrad der Linse angepasst. Die Krümmung ist vorzugsweise genügend lang ausgebildet, um einen wesentlichen Teil der vertikalen Höhe der beiden Schenkel der gefalteten Linse zu kontaktieren. Zudem ist die Kolbenspitze breit genug ausgebildet, damit sie beide Schenkel der gefalteten Linse kontaktiert.

In einem bevorzugten Ausführungsbeispiel weist die Kolbenspitze eine Einbuchtung auf zur Aufnahme eines Haptic's der Linse, um eine Beschädigung des Haptic's zu verhindern. Weist zudem die Kolbennadel im Bereich hinter der Kolbenspitze eine Auflagefläche auf, welche mit dem tiefsten Bereich dieser Einbuchtung fluchtet, so wird die Sicherheit noch erhöht.

Weitere vorteilhafte Ausführungsformen gehen aus den abhängigen Patentansprüchen hervor.

### Kurze Beschreibung der Zeichnung

Im folgenden wird der Erfindungsgegenstand anhand von bevorzugten Ausführungsbeispielen, welche in der beiliegenden Zeichnung dargestellt sind, erläutert. Es zeigen:
- Figur 1a: eine perspektivische Darstellung eines Injektors;
- Figur 1b: den Injektor gemäss Figur 1a mit einer durchgestossenen erfindungsgemässen Kolbennadel;
- Figur 2a: eine perspektivische Darstellung einer erfindungsgemässen Kolbennadel mit einer Kolbenspitze gemäss einer ersten Ausführungsform;
- Figur 2b: die Kolbennadel gemäss Figur 2a in einer Seitenansicht;
- Figur 2c: ein Teil der Kolbennadel gemäss Figur 2a in einer vergrösserten Darstellung;
- Figur 2d: eine Ansicht der Kolbennadel gemäss Figur 2a von vorne;
- Figur 3a: eine perspektivische Darstellung des vorderen Bereichs einer erfindungsgemässen Kolbennadel gemäss einer zweiten Ausführungsform gemeinsam mit einer gefalteten Linse;
- Figur 3b: die Kolbennadel gemäss Figur 3a in einer vergrösserten Seitenansicht;
- Figur 3c: eine Ansicht der Kolbennadel gemäss Figur 3a von vorne;

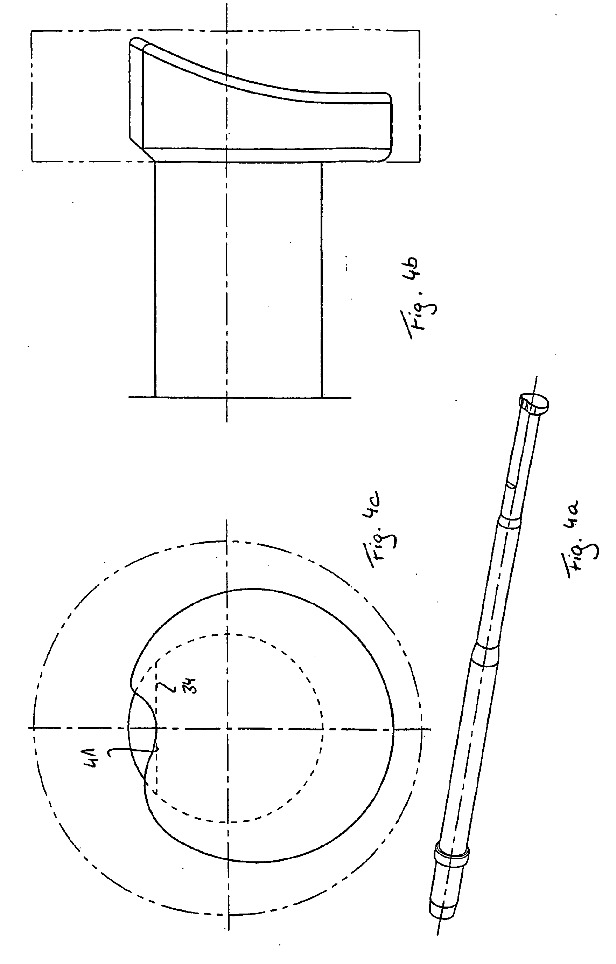

- Figur 4a: eine Seitenansicht einer erfindungsgemässen Kolbennadel mit einer Kolbenspitze gemäss einer dritten Ausführungsform;
- Figur 4b: ein Teil der Kolbennadel gemäss Figur 4a in einer vergrösserten Darstellung;
- Figur 4c: eine Ansicht der Kolbennadel gemäss Figur 4a von vorne;

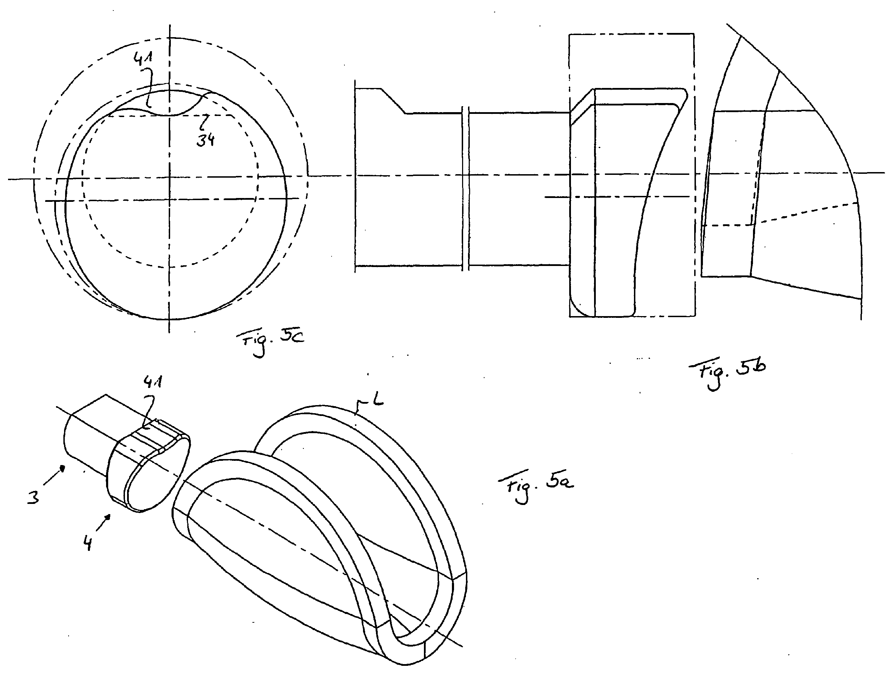

- Figur 5a: eine Seitenansicht einer erfindungsgemässen Kolbennadel mit einer Kolbenspitze gemäss einer vierten Ausführungsform;
- Figur 5b: ein Teil der Kolbennadel gemäss Figur 5a in einer vergrösserten Darstellung;
- Figur 5c: eine Ansicht der Kolbennadel gemäss Figur 5a von vorne;
- Figur 6a: eine perspektivische Darstellung einer erfindungsgemässen Kolbennadel mit einer Kolbenspitze gemäss einer fünften Ausführungsform;
- Figur 6b: die Kolbennadel gemäss Figur 6a in einer Seitenansicht;
- Figur 6c: ein Teil der Kolbennadel gemäss Figur 6a in einer vergrösserten Darstellung;
- Figur 6d: eine Ansicht der Kolbennadel gemäss Figur 6a von vorne;
- Figur 7a: eine perspektivische Darstellung einer erfindungsgemässen Kolbennadel mit einer Kolbenspitze gemäss einer sechsten Ausführungsform;
- Figur 7b: die Kolbennadel gemäss Figur 7a in einer Seitenansicht;
- Figur 7c: ein Teil der Kolbennadel gemäss Figur 7a in einer vergrösserten Darstellung;
- Figur 7d: eine Ansicht der Kolbennadel gemäss Figur 7a von vorne;
- Figur 8a: eine Seitenansicht einer erfindungsgemässen Kolbennadel mit einer Kolbenspitze gemäss einer siebten Ausführungsform;
- Figur 8b: ein Teil der Kolbennadel gemäss Figur 8a in einer vergrösserten Darstellung;
- Figur 8c: eine Ansicht der Kolbennadel gemäss Figur 8a von vorne;
- Figur 9a: eine Seitenansicht einer erfindungsgemässen Kolbennadel mit einer Kolbenspitze gemäss einer achten Ausführungsform;
- Figur 9b: ein Teil der Kolbennadel gemäss Figur 9a in einer vergrösserten Darstellung und
- Figur 9c: eine Ansicht der Kolbennadel gemäss Figur 9a von vorne.

### Wege zur Ausführung der Erfindung

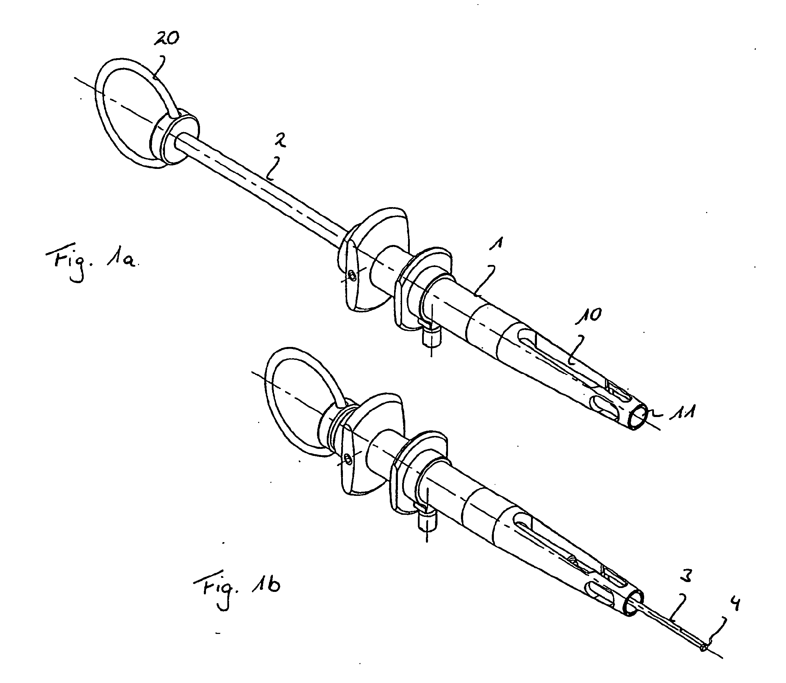

In den Figuren 1a und 1b ist exemplarisch ein intraokularer Injektor dargestellt. Die nachfolgend beschriebenen Ausführungsformen der erfindungsgemässen Kolbennadel lassen sich jedoch auch in anderen, insbesondere den heute handelsüblichen Injektoren verwenden.

Der Injektor weist einen hülsenförmigen Griffkörper 1 auf, welcher von einem Kolben durchsetzt ist. Der Kolben weist eine Kolbenstange 2 mit einem an seinem hinteren Ende angebrachten Fingerring 20, insbesondere für den Daumen, und einer an seinem vorderen Ende angebrachten Kolbennadel 3 auf. Die Kolbennadel 3 kann lösbar an der Kolbenstange 2 befestigt sein, insbesondere über eine Gewindeverbindung.

Der Griffkörper weist eine Linsenaufnahme 10 zur Aufnahme einer hier nicht dargestellten Linse auf. Die Linse wird üblicherweise mit einer Kartusche in die Linsenaufnahme 10 eingelegt. Dabei ragt die Kartusche aus dem vorderen Ende der Linsenaufnahme heraus. Die Linse wird mittels des Kolbens aus der Kartusche gestossen und in ein Auge eines Patienten injiziert.

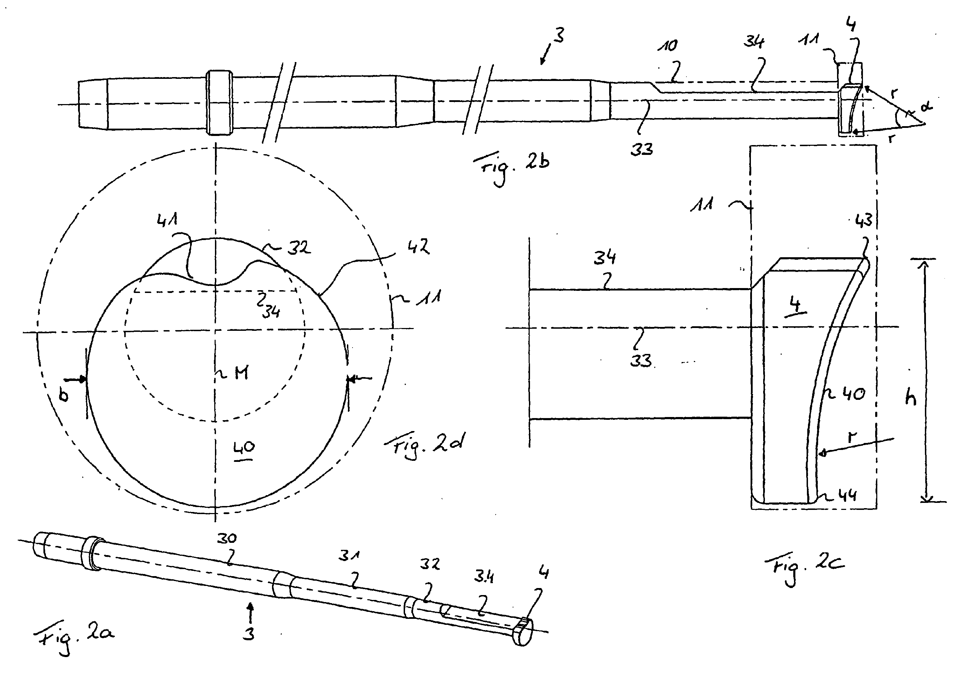

In den Figuren 2a bis 2d ist ein erstes Ausführungsbeispiel einer erfindungsgemässen Kolbennadel 3 dargestellt. Die Kolbennadel 3 ist in diesen Figuren wie auch in allen nachfolgend beschriebenen Figuren vergrössert gezeichnet.

Die Kolbennadel 3 ist vorzugsweise aus Metall, insbesondere Titan, gefertigt. Andere Materialien sind jedoch auch einsetzbar. Sie verjüngt sich vorzugsweise zu ihrem vorderen Ende hin. In diesem Beispiel ist sie mehrstufig ausgebildet, wobei hier drei Stufen 30, 31, 32 vorhanden sind. Am genannten vorderen Ende ist eine Kolbenspitze 4 angeformt, welche gegenüber einer Längsachse 33 der Kolbennadel 3 axial versetzt ist. Die Kolbenspitze 4 ist breiter ausgebildet als ein an sie anschliessender Bereich der Kolbennadel 3 und bildet somit einen Schuh. Dieser anschliessende Bereich weist vorzugsweise eine plane Facette auf, welche eine Auflagefläche 34 bildet.

Der in den Figuren 2b und 2c gestrichelt dargestellte Körper stellt den äusseren Griffkörper 1, beziehungsweise sein vorderes Ende mit der Linsenaufnahme 10 und der Durchlassöffnung 11 dar. Wie in Figur 2b erkennbar ist, dient die zweite Stufe 31 als vorderer Anschlag beim Vorwärtsstossen des Kolbens.

Der in Figur 2d gestrichelt dargestellte äusserste Kreis stellt die äusseren Umrisse der vordersten Spitze des Griffkörpers 1 dar, welche die Durchlassöffnung 11 beinhaltet.

Die Kolbenspitze 4 ist vorzugsweise ebenfalls aus einem Metall, insbesondere Titan, gefertigt. Sie kann jedoch auch aus einem anderen Material bestehen oder beschichtet sein. Sie weist an einer vorderen Stirnfläche 40 einen Kontaktierungsbereich auf. Mit diesem Kontaktierungsbereich wird die gefaltete Linse kontaktiert und nach vorne gestossen. Diese Stirnfläche 40 ist zylindrisch gekrümmt. Das heisst, dass die vordere Spitze in der Seitenansicht gemäss den Figuren 2b und 2c gekrümmt ausgebildet ist, in der Ebene senkrecht zu dieser Zeichnungsebene jedoch plan verläuft. Es ist auch möglich, lediglich den Randbereich entsprechend zu gestalten und einen inneren, zentralen Bereich der Stirnfläche mit einer anderen Form zu versehen. Die vordere Stirnfläche 40 der Kolbenspitze 4 weist somit einen ersten Randbereich 43 auf, welcher der Kolbennadel 3 weiter vorsteht als ein diametral gegenüberliegender zweiter Randbereich 44. Im hier dargestellten Beispiel liegt dieser erste Randbereich näher an der Längsachse 33 der Kolbennadel 3 als der zweite Randbereich 44.

Der Krümmungsradius r der Stirnfläche der Kolbenspitze 4 ist dem Radius der Linse angepasst und beträgt üblicherweise 3 mm.

Der Öffnungswinkel α liegt üblicherweise zwischen 21° und 29°. Die maximale Breite b beträgt üblicherweise 1,6 - 2,2 mm. Der Öffnungswinkel α und die maximale Breite b tragen dem Faltungsgrad der Linse Rechnung. Die maximale Höhe h beträgt üblicherweise 1,7 - 2,2 mm. Es ist jedoch auch möglich, die Stirnfläche 40 nicht kreiszylindrisch gekrümmt sondern allgemein zylindrisch gekrümmt, beispielsweise mit einer elliptischen Zylinderfläche, auszubilden.

Wie in den Figuren 2a und 2d erkennbar ist, weist die Kolbenspitze 4 eine Mantelfläche 42 auf, welche eine Einbuchtung 41 besitzt. Die Einbuchtung 41 ist am ersten Randbereich 43 angeordnet und ist bezüglich einer axialen Mittelebene M asymmetrisch geformt. Diese Einbuchtung 41 dient zur Aufnahme mindestens eines, vorzugsweise genau eines Haptic's der Linse. Dieser Haptic kann zudem auf der Auflagefläche 34 der Kolbennadel 3 aufliegen, so dass er sich nicht zwischen Kolbennadel 3 und innerer Wandung des Griffkörpers 1 verheddern kann. Vorzugsweise fluchtet deshalb der unterste Bereich der Einbuchtung 41 mit der Auflagefläche 34. Im hier dargestellte Ausführungsbeispiel liegt jedoch die Auflagefläche 34 etwas tiefer als die Einbuchtung 41, wie in Figur 2d ersichtlich ist.

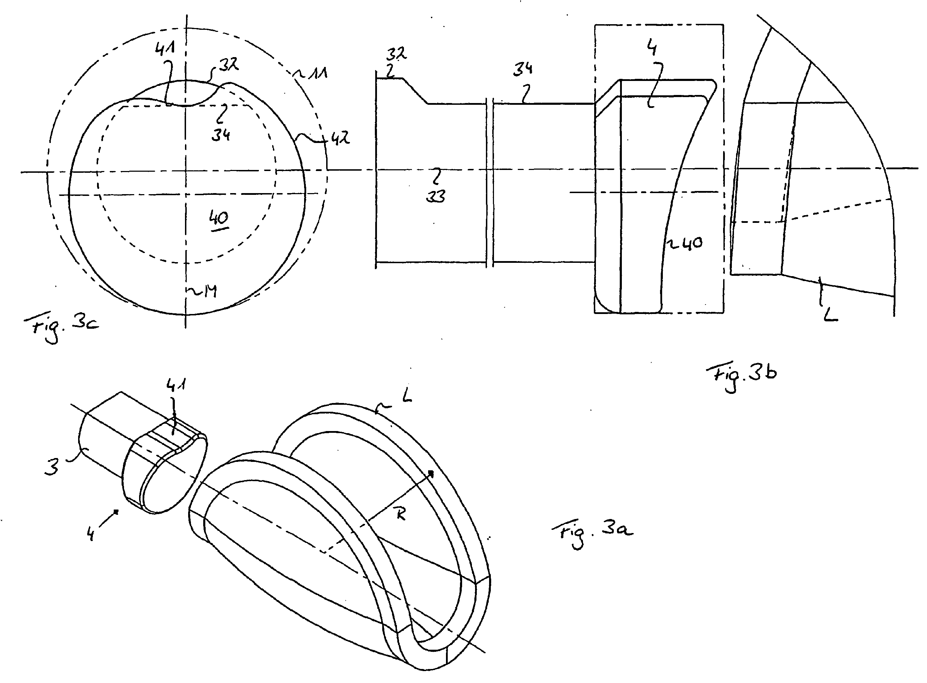

In den Figuren 3a bis 3c ist ein zweites Ausführungsbeispiel dargestellt. Bevor darauf eingegangen wird, sei vermerkt, dass hier nun die gefaltete Linse L in Relation zur Kolbenspitze 4 dargestellt ist. Wie insbesondere in Figur 3b erkennbar ist, entspricht der Krümmungsradius r der Stirnfläche 40 der Kolbenspitze 4 mindestens annähernd dem Radius R der Linse L. Dadurch wird eine optimale Anlage gewährleistet.

Diese Linsenradien R können je nach Herstellerfirma und Linsentyp variieren. Zudem sind die Haptic's unterschiedlich geformt. Oft wird für einen bestimmten Injektor ein bestimmter Linsentyp empfohlen. Um optimale Resultate zu erzielen, ist deshalb der Krümmungsradius r und der Öffnungswinkel α der Kolbenspitze 3 sowie vorzugsweise auch die Form der Einbuchtung 41 entsprechend angepasst. Ebenso können die verwendeten Kartuschen je nach Injektor und Linsentyp eine andere Form aufweisen. Deshalb ist die Kolbenspitze 3 entsprechend optimiert, in dem sie je nach Kartuschentyp nuanciert anders geformt ist. So weicht sie je nach Kartuschentyp und Injektortyp in geringen Massen von ihrer kreisförmigen Mantellinien-Grundform ab und Lage und Form ihrer Einbuchtung 41 ist entsprechend angepasst.

In den weiteren Figuren sind weitere Ausführungsbeispiele dargestellt, welche jeweils handelsüblichen Injektoren, Linsen und Kartuschen angepasst sind. Die in diesen und nachfolgenden Figuren angegebenen Ausführungsbeispiele weisen vorzugsweise ähnliche Masse auf wie das erste Ausführungsbeispiel.

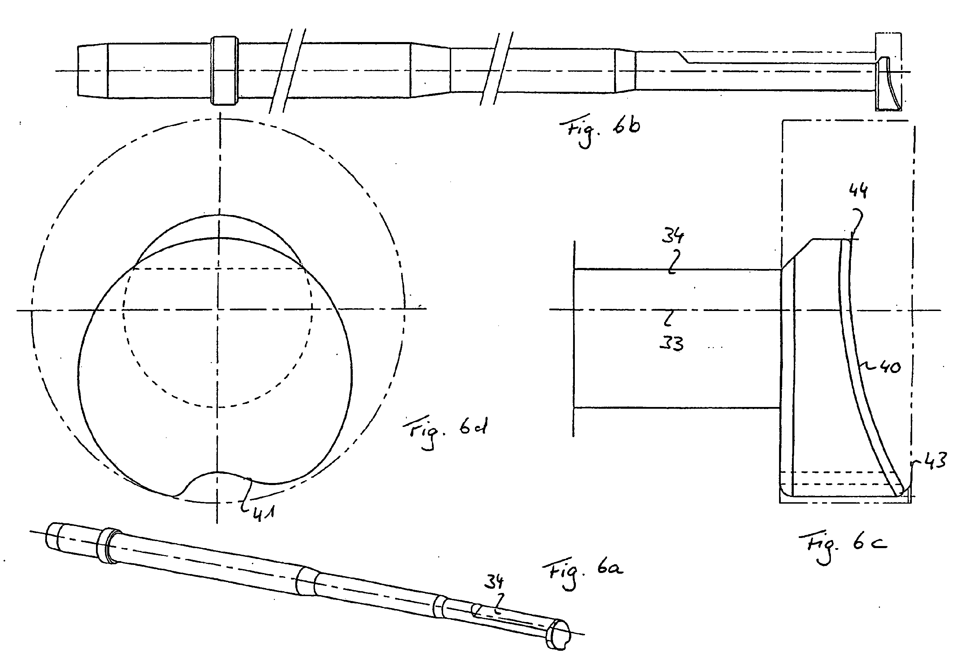

Im Ausführungsbeispiel gemäss den Figuren 6a bis 6d ist der erste Randbereich 43 weiter entfernt von der Längsachse 33 als der zweite Randbereich 44. Die Einbuchtung 41 ist trotzdem im ersten Randbereich 43 angeordnet. Die Auflagefläche 34 dient zur besseren Durchführung durch die Kartusche.

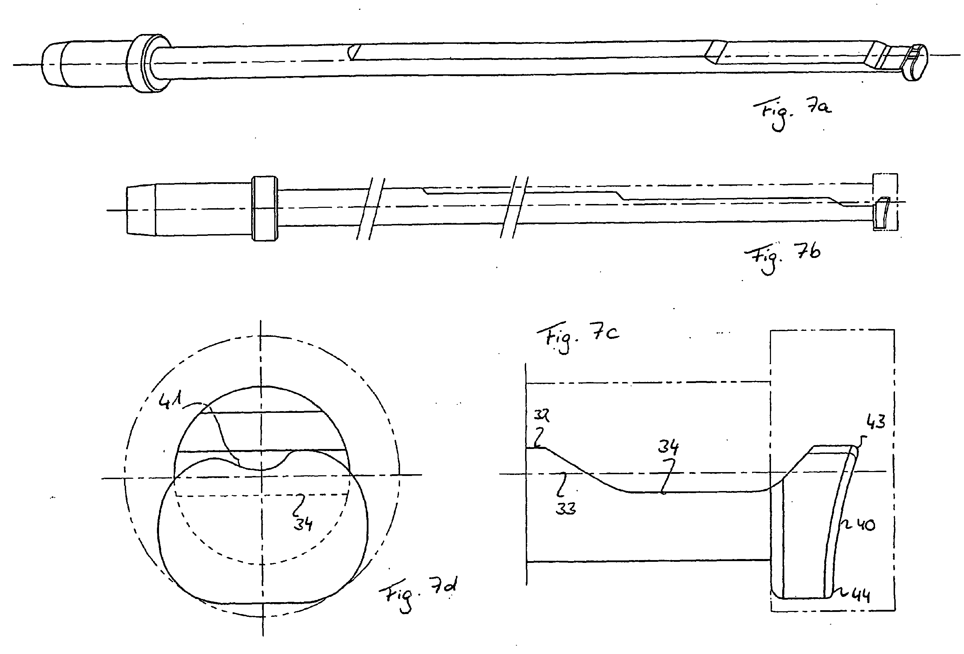

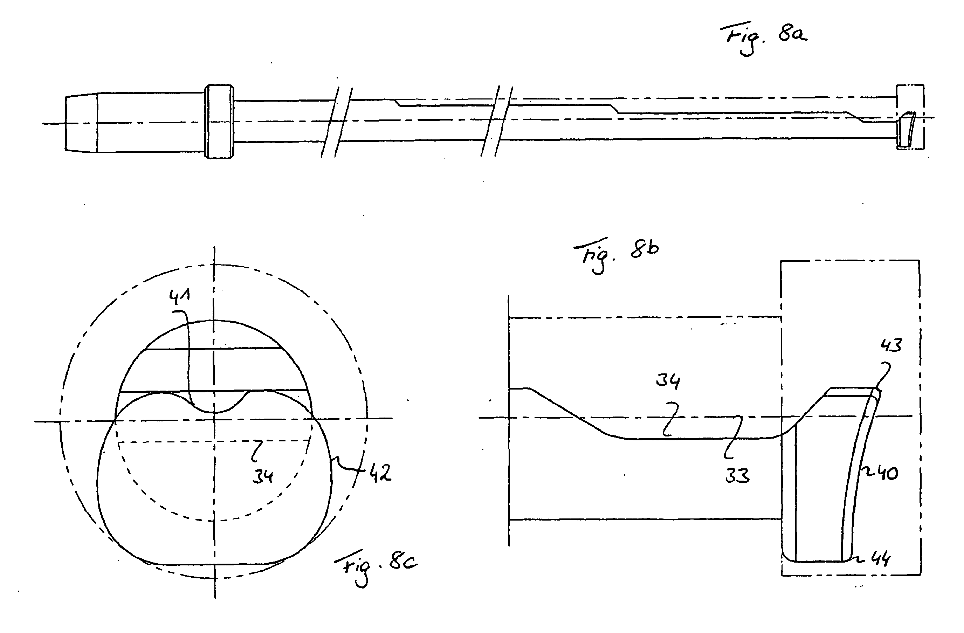

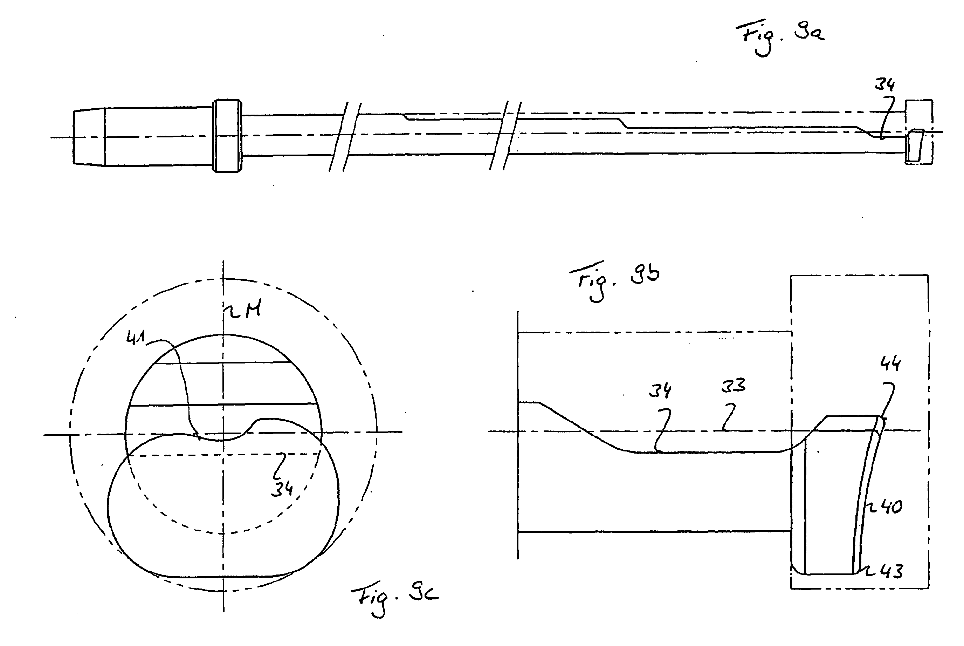

In den Ausführungsbeispielen gemäss den Figuren 7a bis 7d und 9a bis 9c weist die Kolbenspitze 4 eine eher abgeflacht kreisförmige oder elliptische Grundform auf. Im Ausführungsbeispiel gemäss den Figuren 8a bis 8c ist die Einbuchtung 41 mindestens annähernd symmetrisch zur Mittelebene M ausgebildet und die Grundform ist wiederum eher abgeflacht kreisförmig. In diesen Ausführungsbeispielen weist zudem die Kolbennadel drei abgeflachte Facetten auf. Die erste Facette, welche wiederum die Auflagefläche 34 bildet, ist dabei wesentlich tiefer angeordnet als der unterste Bereich der Einbuchtung 41.

Die erfindungsgemässe Kolbennadel 3 mit ihrer speziell geformten Kolbenspitze 3 ermöglicht eine sichere Führung der Linse L beim Injizieren in ein Auge und verhindert ein Verklemmen der Linse im Injektor. Zudem ist sie auf einfache Art und Weise herstellbar.

### Bezugszeichenliste

- 1: Griffkörper
- 10: Linsenaufnahme
- 11: Durchlassöffnung
- 2: Kolbenstange
- 20: Fingerring
- 3: Kolbennadel
- 30: Erste Stufe
- 31: Zweite Stufe
- 32: Dritte Stufe
- 33: Längsachse
- 34: Auflagefläche
- 4: Kolbenspitze
- 40: vordere Stirnfläche
- 41: Einbuchtung
- 42: Mantelfläche
- 43: Erster Endbereich
- 44: Zweiter Endbereich

- r: Krümmungsradius der Stirnfläche der Kolbenspitze
- α: Öffnungswinkel
- b: maximale Breite
- h: maximale Höhe
- L: Linse
- R: Krümmungsradius der Linse
- M: axiale Mittelebene

## Patentansprüche

1. Kolbennadel (3) für einen intraokularen Linsen-Injektor, wobei die Kolbennadel (3) eine Kolbenspitze (4) mit einem Kontaktierungsbereich zur Kontaktierung einer Linse (L) aufweist, wobei der Kontaktierungsbereich in einer vorderen Stirnfläche (40) der Kolbenspitze (4) angeordnet ist, **dadurch gekennzeichnet, dass** mindestens der Kontaktierungsbereich der vorderen Stirnfläche (40) derartig zylinderförmig gekrümmt ist, dass dieser ein negatives Abbild einer Mantelfläche eines Zylinders bildet und einen Öffnungswinkel (α) von 21° bis 39° aufweist.

2. Kolbennadel nach Anspruch 1, **dadurch gekennzeichnet, dass** der Kontaktierungsbereich einen Krümmungsradius (r) aufweist, der mindestens annähernd dem Krümmungsradius (R) einer Intraokularlinse (L) entspricht, insbesondere von annähernd 3 mm.

3. Kolbennadel nach Anspruch 1 oder 2, **dadurch gekennzeichnet, dass** die Kolbenspitze (4) eine Mantellinie mit im wesentlichen kreisförmiger, abgeflacht kreisförmiger oder elliptischer Grundform aufweist.

4. Kolbennadel nach einem der Ansprüche 1 bis 3, **dadurch gekennzeichnet, dass** die Kolbenspitze (4) eine Mantelfläche (42) mit einer Einbuchtung (41) zur Aufnahme mindestens eines Haptic's der Linse (L) aufweist.

5. Kolbennadel nach Anspruch 4, **dadurch gekennzeichnet, dass** die Kolbennadel (3) in einem Bereich hinter der Kolbenspitze (4) eine Auflagefläche (34) aufweist und **dass** die Einbuchtung (41) in ihrem tiefsten Bereich mit der Auflagefläche (34) fluchtet.

6. Kolbennadel nach einem der Ansprüche 1 bis 5, **dadurch gekennzeichnet, dass** die Stirnfläche (40) der Kolbenspitze (4) mit einem ersten Randbereich (43) weiter der Kolbennadel (3) vorsteht als an einem zweiten, diametral gegenüberliegenden zweiten Randbereich (44).

7. Kolbennadel nach den Ansprüchen 5 und 6, **dadurch gekennzeichnet, dass** die Einbuchtung (41) am ersten Randbereich (43) angeordnet ist.

8. Kolbennadel nach einem der Ansprüche 1 bis 7, **dadurch gekennzeichnet, dass** die Kolbennadel (3) eine Längsachse (33) aufweist und dass die Kolbenspitze (4) axial versetzt zu dieser Längsachse (33) angeordnet ist.

9. Kolbennadel nach den Ansprüchen 6 und 8, **dadurch gekennzeichnet, dass** der erste Randbereich (43) derjenige Randbereich ist, welcher näher an der Längsachse (33) liegt als der zweite Randbereich (44) oder dass er derjenige Randbereich ist, welcher weiter von der Längsachse (33) entfernt angeordnet ist als der zweite Randbereich (44).

10. Kolbennadel nach einem der Ansprüche 1 bis 9, **dadurch gekennzeichnet, dass** die Kolbenspitze (4) breiter ausgebildet ist als ein anschliessender Bereich (32) der Kolbennadel (3).

11. Injektor zum Einsetzen einer intraokularen Linse in ein Auge, umfassend eine Kolbennadel nach einem der Ansprüche 1 bis 10.

12. Injektor nach Anspruch 11, mit einer Durchlassöffnung (11) für die Linse (L), um die Linse in das Auge einzusetzen, wobei der Injektor dazu ausgebildet ist, eine Linse aufzunehmen, deren Krümmungsradius (R) grösser ist als der Radius der Durchlassöffnung (11), und wobei der Radius (r) des Kontaktierungsbereichs der Kolbenspitze (4) wenigstens annähernd dem Krümmungsradius (R) der Linse (L) entspricht.

## Claims

1. A plunger needle (3) for an intraocular lens injector, the plunger needle (3) having a plunger tip (4) with a contact area for making contact with a lens (L), the contact area being arranged in a front face (40) of the plunger tip (4), **characterised in that** at least the contact area of the front face (40) is cylindrically curved in a manner to form a negative image of a jacket surface of a cylinder and to have an angle of opening (α) of 21° to 39°.

2. The plunger needle according to claim 1, **characterised in that** the contact area has a radius of curvature (r) at least approximately matching the radius of curvature (R) of an intraocular lens (L), in particular a radius of curvature of approximately 3 mm.

3. The plunger needle according to claim 1 or 2, **characterised in that** the plunger tip (4) has a circumference line having a substantially circular, flattened circular or elliptical basic shape.

4. The plunger needle according to one of claims 1 to 3, **characterised in that** the plunger tip (4) has a jacket surface (42) with an indentation (41) for receiving at least one haptic of the lens (L).

5. The plunger needle according to claim 4, **characterised in that** the plunger needle (3) has a bearing surface (34) in an area behind the plunger tip (4), and that the indentation (41) in its deepest area is flush with the bearing surface (34).

6. The plunger needle according to one of claims 1 to 5, **characterised in that** the front face (40) of the plunger tip (4), at a first edge area (43), protrudes farther from the plunger needle (3) than at a diametrically opposite second edge area (44).

7. The plunger needle according to claims 5 and 6, **characterised in that** the indentation (41) is arranged at the first edge area (43).

8. The plunger needle according to one of claims 1 to 7, **characterised in that** the plunger needle (3) has a longitudinal axis (33), and that the plunger tip (4) is arranged axially offset relative to this longitudinal axis (33).

9. The plunger needle according to claims 6 and 8, **characterised in that** the first edge area (43) is the edge area which lies nearer the longitudinal axis (33) than the second edge area (44), or that it is the edge area arranged farther away from the longitudinal axis (33) than the second edge area (44).

10. The plunger needle according to one of claims 1 to 9, **characterised in that** the plunger tip (4) is wider than an adjacent area (32) of the plunger needle (3).

11. An injector for inserting an intraocular lens into an eye, comprising a plunger needle according to one of claims 1 to 10.

12. The injector according to claim 11, having a through-opening (11) for the lens (L) for inserting the lens into the eye, the injector being adapted to receive a lens whose radius of curvature (R) is larger than the radius of the through-opening (11), and the radius (r) of the contact area of the plunger tip (4) matching at least approximately the radius of curvature (R) of the lens (L).

## Revendications

1. Tige de piston (3) pour injecteur intraoculaire de lentille, la tige de piston (3) présentant une pointe de piston (4) dotée d'une zone de contact destinée à assurer le contact avec une lentille (L), la zone de contact étant disposée dans une partie frontale avant (40) de la pointe de piston (4), **caractérisée en ce qu'**au moins la zone de contact de la surface frontale avant (40) a une courbure cylindrique qui forme une image négative de la surface d'enveloppe d'un cylindre et présente un angle d'ouverture (α) de 21 ° à 39 °.

2. Tige de piston selon la revendication 1, **caractérisée en ce que** la zone de contact a un rayon de courbure (r) qui correspond au moins approximativement au rayon de courbure (R) d'une lentille intraoculaire (L) et en particulier à approximativement 3 mm.

3. Tige de piston selon les revendications 1 ou 2, **caractérisée en ce que** la pointe de piston (4) a une surface d'enveloppe dont la forme de base est essentiellement circulaire, circulaire aplatie ou elliptique.

4. Tige de piston selon l'une des revendications 1 à 3, **caractérisée en ce que** la pointe de piston (4) a une surface d'enveloppe (42) dotée d'un creux (41) qui reprend au moins une partie de saisie pour un élément haptique de la lentille (L).

5. Tige de piston selon la revendication 4, **caractérisée en ce que** la tige de piston (3) présente une surface de pose (34) dans une zone située derrière la pointe de piston (4) et **en ce que** la partie la plus profonde du creux (41) est alignée sur la surface de pose (34).

6. Tige de piston selon l'une des revendications 1 à 5, **caractérisée en ce qu'**une première bordure (43) de la surface frontale (40) de la pointe de piston (4) déborde plus loin de la tige de piston (3) qu'une deuxième bordure (44) diamétralement opposée.

7. Tige de piston selon les revendications 5 et 6, **caractérisée en ce que** le creux (41) est disposé sur la première bordure (43).

8. Tige de piston selon l'une des revendications 1 à 7, **caractérisée en ce que** la tige de piston (3) présente un axe longitudinal (33) et **en ce que** la pointe de piston (4) est disposée à un décalage axial par rapport à cet axe longitudinal (33).

9. Tige de piston selon les revendications 6 et 8, **caractérisée en ce que** la première bordure (43) est la bordure située plus près de l'axe longitudinal (33) que la deuxième bordure (44) ou la bordure située plus loin de l'axe longitudinal (33) que la deuxième bordure (44) .

10. Tige de piston selon l'une des revendications 1 à 9, **caractérisée en ce que** la pointe de piston (4) est plus large qu'une partie (32) adjacente de la tige de piston (3).

11. Injecteur destiné, à placer une lentille intraoculaire dans un oeil, qui comprend une tige de piston selon l'une des revendications 1 à 10.

12. Injecteur selon la revendication 11, qui présente une ouverture (11) de passage de lentille (L) pour placer la lentille dans l'oeil, l'injecteur étant configuré de manière à reprendre une lentille dont le rayon de courbure (R) est plus grand que le rayon de l'ouverture de passage (11), le rayon (r) de la zone de contact de la pointe de piston (4) correspondant au moins approximativement au rayon de courbure (R) de la lentille (L).
